# EUROPÄISCHE PATENTSCHRIFT

(11) **EP 0 792 877 B1**
(45) Veröffentlichungstag und Bekanntmachung des Hinweises auf die Patenterteilung: **12.03.2003**
(21) Anmeldenummer: 96121010.1
(22) Anmeldetag: 31.12.1996
(51) Int. Cl.: C07D 307/32

(54) **Verfahren zur Herstellung von 2-Acetyl-gamma-butyrolacton**
Process for the preparation of 2-acetyl-gamma-butyrolactone
Procédé pour la préparation de 2-acetyl-gamma-butyrolactone

(30) Priorität: 24.02.1996 DE 19606975
(43) Veröffentlichungstag der Anmeldung: 03.09.1997
(73) Patentinhaber: Degussa AG, 40474 Düsseldorf (DE)
(72) Erfinder: Köhler, Günther, Dr., 45770 Marl (DE); Uhlenbrock, Wilfried, Dr., 45770 Marl (DE)

(56) Entgegenhaltungen:
- CHEMICAL ABSTRACTS, vol. 099, no. 17, 24.Oktober 1983 Columbus, Ohio, US; abstract no. 139770, ".alpha.-Acetyllactones" XP002031932 & JP 58 099 473 A (DAINIPPON INK AND CHEMICALS, INC.;JAPAN) 13.Juni 1983
- CHEMICAL ABSTRACTS, vol. 122, no. 11, 13.März 1995 Columbus, Ohio, US; abstract no. 132878, ZHOU J ET AL: "Improved synthesis of.alpha.-acetyl-.gamma.-butyrolactone" XP002031933 & ZHONGGUO YIYAO GONGYE ZAZHI (ZYGZEA,10018255);94; VOL.25 (10); PP.461, FUDAN UNIV.;DEP. CHEM.; SHANGHAI; 200433; PEOP. REP. CHINA (CN),
- CHEMICAL ABSTRACTS, vol. 120, no. 24, 13.Juni 1994 Columbus, Ohio, US; abstract no. 301546, WU Y ET AL: "Production of.alpha.-acetylbutyrolactone from.gamma.-butyrolactone" XP002031934 & NANJING HUAGONG XUEYUAN XUEBAO (NAXUEI,10005994);92; VOL.14 (3); PP.48-52, NANJING INST. CHEM. TECHNOL.;DEP. CHEM. ENG.; NANJING; PEOP. REP. CHINA (CN),
- CHEMICAL ABSTRACTS, vol. 111, no. 13, 25.September 1989 Columbus, Ohio, US; abstract no. 114977, LIPKIN M A ET AL: "Characteristics of.gamma.-butyrolactone and ethyl acetate condensation" XP002031935 & KHIM.-FARM. ZH. (KHFZAN,00231134);88; VOL.22 (12); PP.1465-9, NPO "VITAMINY";MOSCOW; USSR (SU),

## Beschreibung

Die Erfindung betrifft ein Verfahren zur Herstellung von 2-Acetyl-gamma-butyrolacton durch kontinuierliche Umsetzung von gamma-Butyrolacton mit einem Essigsäureester in einer Kondensationsreaktion unter Mitwirkung eines stark basischen Mittels. 2-Acetyl-gamma-butyrolacton ist ein wertvolles Zwischenprodukt für die Herstellung von Wirkstoffen für Arzneimittel und für Pflanzenschutzmittel sowie von verschiedenen heterocyclischen Verbindungen.

Die Herstellung von 2-Acetyl-gamma-butyrolacton durch eine Kondensationsreaktion von Butyrolacton mit Essigsäureestern und anschließende Protonierung (oder Neutralisierung) des zunächst entstehenden Enolats ist grundsatzlich bekannt; die Reaktion wird durch das folgende allgemeine Formelschema wiedergegeben:

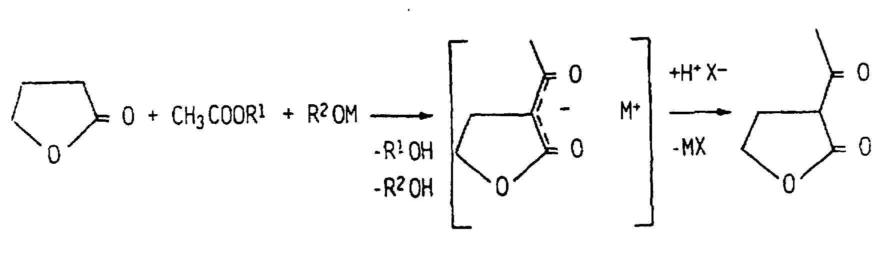

In den Formeln bedeuten R¹ und R² gleiche oder verschiedene niedere Alkylreste, bedeutet M ein Alkalimetall- oder ein quaternäres Ammoniumion und steht X für einen Säurerest und X- für ein Saureanion

Die Acetylierung von gamma-Butyrolacton mit Essigsäureestern in Gegenwart von stark basischen Stoffen, wie Natrium, Kalium, Natriumamid, Natriumhydrid oder Alkalialkoholaten, wurde von F. Korte in Angewandte Chemie, 71, 23, 709-752 (1959) beschrieben. Nach der offengelegten japanischen Patentanmeldung 45/009 538 verwendet man speziell Butylacetat als Essigsäureester und Natriumbutylat als Alkalialkoholat

M.A.Lipkin et al., Khim.-Farm,Zh.,22(12), 1465-1469 empfehlen dagegen Ethylacetat sowie Natriummethylat für ihr absatzweises Verfahren und geben Ausbeuten von 75%d.Th. an. Nach der offengelegten japanischen Patentanmeldung 58/099 473 läßt sich die Ausbeute bei Verwendung dieser Stoffe durch Mitverwendung zusätzlicher, stark polarer Lösungsmittel, wie Dimethylformamid oder Dimethylacetamid, auf 80-85%d.Th. verbessern. In anderen Schriften, wie der polnischen Patentschrift 157 263 oder der offengelegten japanischen Patentanmeldung 58/162 585, werden an Stelle von Essigsäureestern Acylhalogenide als Acylierungsmittel empfohlen. Dabei sind jedoch die Ausbeuten niedrig, und es fallen chlorierte organische Nebenprodukte an, die durch Destillation nur schwierig abtrennbar sind. Darüber hinaus sind die Kosten für die Einsatzstoffe höher als bei den Verfahren, die mit den wohlfeilen Essigsäureestern arbeiten.

Es wurde nun gefunden, daß sich 2-Acetyl-gamma-butyrolacton aus gamma-Butyrolacton und einem Essigsäureester in einer Kondensationsreaktion unter Mitwirkung eines stark basischen Stoffs mit nachfolgender Protonierung des zunächst entstehenden Enolats vorteilhaft herstellen laßt, wenn man das gamma-Butyrolacton, den Essigsaureester und den stark basischen Stoff in einem Verhältnis von 1,0 bis 6.0 Molteilen Essigsaureester und 0,9 bis 1,6 Molteilen des stark basischen Stoffs je Molteil gamma-Butyrolacton kontinuierlich einer Reaktionszone zufuhrt, in der die Kondensationsreaktion stattfindet und aus der anteilsweise oder kontinuierlich Reaktionsgemisch abgezogen und protoniert wird.

Das Verfahren nach der Erfindung ist mit einer Reihe uberraschender Vorteile verbunden. Man erhält das 2-Acetyl-gamma-butyrolacton nach einfacher Destillation in hoher Reinheit von >99% und mit bisher unerreichten, sehr guten Ausbeuten von über 90%, bezogen auf eingesetztes gamma-Butyrolacton, das also hochselektiv umgesetzt wird. Neben- und bzw. oder Folgereaktionen, die die Selektivität und die Ausbeute an 2-Acetyl-gammabutyrolacton herabsetzen, werden weitgehend zuruckgedrangt So fallen z.B. Hydroxy- oder Alkoxybuttersäurederivate, die nicht nur die Ausbeute mindern, sondern zudem durch Destillation schwierig von 2-Acetyl-gamma-butyrolacton abzutrennen sind, in erheblich geringeren Mengen an als bei den Verfahren nach dem Stand der Technik.

Die Selektivität und die Ausbeute sind überraschenderweise besser als bei Mitverwendung von zusätzlichen polaren Lösungsmitteln nach der erwähnten offengelegten japanischen Patentanmeldung 58/099 473. Ein zusätzliches polares Lösungsmittel bewirkt zudem eine verminderte Raum-Zeit-Ausbeute sowie zusätzlichen Destillationsaufwand, insbesondere wenn Alkalialkoholate als stark basische Kondensationsmittel verwendet werden, die zusätzlichen Alkohol ergeben. Nicht polare, inerte Lösungsmittel, wie Toluol, können dagegen mitverwendet werden, ohne daß dadurch die Ausbeute verschlechtert wird.

Das Verfahren nach der Erfindung verbraucht weniger Energie und ist regelungstechnisch einfacher als die absatzweisen Verfahren des Standes der Technik. Es wurde gefunden, daß bei der absatzweisen Mischung von gamma-Butyrolacton mit einem Alkoholat eine erhebliche Wärmemenge frei wird. Bei Verwendung von Natriummethylat als stark basischem Stoff wurde diese Wärmemenge mit -55 kJ je Mol gamma-Butyrolacton gemessen. Bei der Herstellung im technischen Maßstab ist die Umstellung von der anfänglich benötigten Kühlung auf die später erforderliche Heizung regel- und sicherheitstechnisch anspruchsvoll, und zudem wird viel Energie vernichtet. Das Verfahren nach der Erfindung nutzt die erwähnte frei werdende Wärme voll aus und ist im stationären Zustand regel- und sicherheitstechnisch einfach und zuverlässig zu beherrschen, da keine Wärme vom System abgegeben wird und nur in geringem Umfang nachgeregelt werden muß.

Schließlich wird die Ausbeute an 2-Acetyl-gamma-butyrolacton weiter verbessert, wenn man bei der Protonierung mittels einer Saure eine Temperatur von -5 bis +50°C und insbesondere einen bestimmten pH-Wert, nämlich von 4 bis 7 einhält. Offenbar führt das stark saure Milieu, dem das 2-Acetyl-gamma-butyrolacton vor dem Erreichen des Neutralpunkts ausgesetzt ist, wenn man ohne besondere Vorsichtsmaßnahmen das Reaktionsgemisch in die Säure einträgt oder die Säure dem Reaktionsgemisch zufügt, zu Hydrolysereaktionen und damit verbundenen betrachtlichen Ausbeuteverlusten.

Für das Verfahren nach der Erfindung eignen sich Ester der Essigsäure mit einwertigen Alkoholen, wie Methanol, Ethanol, 1- und 2-Isopropanol, 1- und 2-Butanol, 2-Methyl-1-propanol, 1-Hexanol, 2-Ethyl-1-hexanol, Benzylalkohol und beta-Phenylethylalkohol. Ester aus Alkanolen mit 1 bis 4 Kohlenstoffatomen werden bevorzugt. Besonders bevorzugt wird Methylacetat. Da der Alkoholrest des Essigsäureesters bei der Kondensationsreaktion als Alkohol abgespalten wird, kann man ohne weiteres auch ein Gemisch von Essigsäureestern einsetzen.

Von den als Kondensationsmitteln bekannten stark basischen Mitteln werden die Alkalialkoholate bevorzugt, insbesondere die Lithium-, Natrium- und Kaliumalkoholate, die sich von Alkanolen mit 1 bis 4 Kohlenstoffatomen ableiten. Besonders bevorzugt werden Natriumethylat und insbesondere Natriummethylat. Andere geeignete stark basische Mittel sind die Alkalimetalle, Alkalimetallhydride und -amide. Statt eines einzigen Stoffs kann man auch ein Gemisch verschiedener geeigneter Stoffe als stark basisches Mittel einsetzen. Das stark basische Mittel kann in feinteiliger Form, z.B. als handelsübliches Natriummethylat-Pulver, mittels üblicher Dosiervorrichtungen, wie Dosierschnecken usw., zugefuhrt werden. Alternativ kann man das feinteilige stark basische Mittel in einer Teilmenge des Essigsäureesters oder in einem inerten, nicht polaren organischen Lösungsmittel, wie Toluol, suspendieren. Gerührte Suspensionen mit 30 bis 60 Gew.% Feststoffgehalt lassen sich oft leichter dosieren als die Feststoffe allein.

Vorteilhaft wendet man das gamma-Butyrolacton, den Essigsäureester und das stark basische Mittel in Mengen von 1.0 bis 5.0, insbesondere von 1.05 bis 2.5 Molteilen Essigsäureester und von 1.0 bis 1.5, insbesondere von 1.05 bis 1.4 Molteilen des stark basischen Mittels je Molteil gamma-Butyrolacton an. Der Anteil des Essigsäureesters kann über die angegebenen Mengen hinaus erhöht werden, wodurch allerdings die Raum-Zeit-Ausbeute vermindert wird. Es ist zweckmäßig, die genannten Reaktionsteilnehmer in möglichst gleichbleibenden, innerhalb der angegebenen Grenzen liegenden molaren Verhältnissen der Reaktionszone zuzufuhren, weil auf diese Weise eine bessere Selektivität erzielt wird

Die Reaktionszone kann der Innenraum eines Rührkesselreaktors sein. Im allgemeinen kann man die Reaktionsparameter Stoffmengen, Reaktionstemperatur und mittlere Verweilzeit so aufeinander abstimmen, daß die Reaktion isotherm und zugleich adiabatisch verläuft. Ein Reaktor mit Vorrichtung zum Heizen (bei langen mittleren Verweilzeiten und/oder kleinen Stoffmengen) und Kühlen (bei kurzen mittleren Verweilzeiten und/oder großen Stoffmengen) ist jedoch zweckmäßig, weil er die erwünschte Flexibilität in der Reaktionsführung sichert. Um die Rückvermischung des zunächst in Form des Alkalienolats vorliegenden 2-Acetylgamma-butyrolactons mit den Ausgangsstoffen gering zu halten, kann man die Reaktionszone unterteilen, indem man mit einer Reaktorkaskade arbeitet. Dabei können in den verschiedenen Reaktoren unterschiedliche (im allgemeinen ansteigende) Temperaturen eingestellt werden. Das Verfahren nach der Erfindung kann auch in einem Strömungsrohr durchgefuhrt werden; bei laminarer Strömung findet praktisch keine Rückmischung statt. Auch dabei läßt sich ein Temperaturgradient einstellen, wenn dies erwünscht ist.

Die optimale Temperatur und die optimale mittlere Verweilzeit in der Reaktionszone bedingen einander wechselseitig. Zweckmaßig liegt die Temperatur im Bereich von 20 bis 160°C, vorteilhaft von 30 bis 140°C Die mittlere Verweilzeit beträgt im allgemeinen 5 Minuten bis 30 Stunden, vorteilhaft 10 Minuten bis 20 Stunden. Für einen gegebenen Essigsäureester, ein gegebenes stark basisches Mittel und eine bestimmte Geometrie der Reaktionszone lassen sich die optimalen Parameter unschwer durch Vorversuche ermitteln.

Das aus der Reaktionszone austretende, 2-Acetyl-gamma-butyrolacton in Form seines Enolats enthaltende Reaktionsgemisch ist bei ca. 50°C im allgemeinen hinreichend dünnflüssig, so daß es durch mechanische Pumpen gefordert werden kann. Bei niedrigeren Temperaturen und/oder einem nur geringen Überschuß an Essigsäureester kann man ein inertes Losungsmittel, wie Toluol, zusetzen, um das Reaktionsgemisch pumpbar einzustellen. Das Reaktionsgemisch wird anteilsweise oder vorteilhaft kontinuierlich aus der Reaktionszone abgezogen.

Das Reaktionsgemisch wird dann mit einer anorganischen Saure, einer organischen Säure oder einem organischen Säureanhydrid protoniert Geeignete anorganische Säuren sind z.B. Halogenwasserstoffsäuren, wie Salzsäure; Schwefelsäure, Phosphorsäure, Salpetersäure und Kohlensaure (als Kohlendioxid). Von den geeigneten organischen Säuren seien z.B. Carbonsäuren, wie Ameisensäure, Essigsäure und Propionsäure, sowie aliphatische oder aromatische Sulfonsäuren, wie Benzol- oder p-Toluolsulfonsäure, genannt. Ein geeignetes organisches Säureanhydrid ist z B. Essigsäureanhydrid. Eine besonders bevorzugte Säure ist Schwefelsäure. Zweckmäßig werden die Säuren mit einem gewissen Anteil an Wasser, z B. etwa 10 bis 70 Gew %, eingesetzt, damit man über die Messung des pH-Wertes eine vollständige Protonierung ohne Säureüberschuß erreicht. Wenn man mit Kohlensäure oder einem organischen Säureanhydrid als Protonierungsmittel arbeitet, sollte eine entsprechende Menge Wasser auf andere Weise zugeführt werden.

Die Protonierung wird vorteilhaft so ausgeführt, daß die Säure und das Reaktionsgemisch gleichzeitig in eine Protonierungszone (oder Neutralisationszone) dosiert werden. Dabei stellt sich in dem entstehenden Gemisch praktisch sofort ein gleichmäßiger pH-Wert ein, der mittels üblicher Meß- und Regeltechnik, vorzugsweise mit Hilfe der Säuredosierung als Regelgröße, in einem Bereich konstant gehalten werden kann, der zweckmäßig von 4 bis 7 und insbesondere von 5 bis 6,5 beträgt. Bei der Protonierung (oder Neutralisation) wird Warme frei, so daß man durch Kühlung dafür sorgen muß, daß die erwünschten Temperaturen im Bereich von -5 bis +50°C. insbesondere von 10 bis 30°C, herrschen

Zum Anfahren der Anlage oder Apparatur legt man zweckmäßig als Grundfullung Essigsaureester oder ein inertes Lösungsmittel vor, führt dann Essigsäureester und gamma-Butyrolacton, getrennt oder im Gemisch, sowie das stark basische Mittel, als Pulver oder Suspension, in den vorgesehenen Mengenverhältnissen zu und beginnt nach einiger Zeit mit der Abnahme von Reaktionsgemisch, während man gleichzeitig die genannten Reaktionsteilnehmer in dem gewünschten Mengenverhältnis weiter zuführt Die zugeführten und die abgenommenen Mengen werden so bemessen, daß der Stand der flüssigen Phase in dem Reaktor oder den Reaktoren gehalten wird

Die folgenden Beispiele werden gegeben, um die Erfindung weiter zu erläutern, nicht jedoch um sie zu limitieren.

### Beispiel 1

In einem 2 l-Glasreaktor mit Feststoffdosiervorrichtung werden 500 ml Ethylacetat als Grundfüllung vorgelegt und auf 55°C erwärmt. Dann werden innerhalb von 60 Minuten über die Feststoffdosiervorrichtung 259 g Natriummethylat-Pulver sowie gleichzeitig über eine Dosierpumpe ein Gemisch aus 344 g gamma-Butyrolacton und 529 g Ethylacetat zudosiert Der Reaktorinhalt wird durch schwaches Heizen auf 55°C gehalten und mit Hilfe eines Flügel rührers gut durchmischt. Dann wird mit der Entnahme von 1.132 g/h Reaktionsgemisch über eine weitere Dosierpumpe begonnen, und gleichzeitig wird die Zuführung von 259 g/h Natriummethylat-Pulver sowie von 344 g/h gamma-Butyrolacton und von 529 g/h Ethylacetat (letztere im Gemisch) fortgesetzt. Dabei bleibt der Stand im Reaktor gleich. Die mittlere Verweilzeit im Reaktor beträgt ca. 60 Minuten Nach wenigen Stunden dieser kontinuierlichen Fahrweise stellt sich ein stationärer Zustand ein. Der Umsatz des gamma-Butyrolactons beträgt nach GC-Analyse ca. 85%, die Selektivitität der Bildung von 2-Acetylgamma-butyrolacton ca. 80%, bezogen auf eingesetztes gamma-Butyrolacton.

Das aus dem Glasreaktor abgezogene Reaktionsgemisch (1.132 g/h) wird in ein druckfestes beheizbares Strömungsrohr als zweiten Reaktor gefordert, in dem durch Heizen eine Temperatur von 90°C aufrechterhalten wird. Infolge des Dampfdrucks der leicht siedenden Komponenten des Reaktionsgemisches stellt sich dabei ein Innendruck von ca. 0.6 MPa ein. Die mittlere Verweilzeit des Gemisches in dem Druckreaktor betragt ca 2 Stunden. Eine dem stündlich zugefuhrten Reaktionsgemisch entsprechende Menge Gemisch wird stündlich als Entnahme abgezogen.

Das aus dem Druckreaktor abgezogene Gemisch (1.132 g/h) sowie 296 g 80% Schwefelsäure werden so in einen Protonierungsreaktor (oder Neutralisierungsreaktor) mit Flügelrührer eindosiert, daß sich ein pH-Wert von 6±0.2 einstellt. Die Menge des Gemisches im Protonierungsreaktor wird so eingeregelt, daß die mittlere Verweilzeit ca. 4 Stunden betragt. Stündlich werden etwa 1.430 g Protonierungsgemisch abgezogen, in dem Natriumsulfat suspendiert ist. Dieses wird durch Filtration abgetrennt und mit etwas Methanol gewaschen. Das Filtrat wird mit der Waschflüssigkeit vereinigt und destilliert, wobei zunächst die Leichtsieder in einem Rotationsverdampfer abgetrennt werden. Bei der Reindestillation über eine mit Raschigringen gefüllte, 20 cm lang Kolonne erhält man 466 g/h gamma-Acetylbutyrolacton, das nach GC-Analyse eine Reinheit von >99% hat. Die Ausbeute beträgt 91% d.Th., bezogen auf eingesetztes gamma-Butyrolacton.

### Beispiel 2

In einem 2 l Glasreaktor mit Feststoffdosiervorrichtung und Rührwerk werden 500 ml Methylacetat als Grundfüllung vorgelegt und auf 45°C erwarmt. Danach werden innerhalb einer Stunde über die Feststoffdosiervorrichtung 194 g Natriummethylat-Pulver sowie gleichzeitig mittels einer Dosierpumpe ein zuvor bereitetes Gemisch von 258 g gamma-Butyrolacton und 422 g Methylacetat zudosiert. Der Reaktorinhalt wird zunächst durch Kühlen auf 45°C gehalten. Nach etwa einer Stunde wird mit dem Abzug von 874 g/h Reaktionsgemisch mit Hilfe einer weiteren Dosierpumpe begonnen, und gleichzeitig wird die Zuführung von 194 g/h Natriummethylat-Pulver, 258 g/h gamma-Butyrolacton und 422 g/h Methylacetat (letztere im Gemisch miteinander) fortgesetzt. Der Stand im Reaktor bleibt konstant, die Verweilzeit beträgt etwa 1 1 h. Nach dem mit dem Abzug von Reaktionsgemisch begonnen wurde, wird die Temperatur des Reaktorinhalts durch schwaches Heizen auf 45°C gehalten.

Das aus dem Reaktor entnommene Reaktionsgemisch wird mit Hilfe der erwahnten zweiten Dosierpumpe kontinuierlich in einen zweiten, 4 l fassenden Stahldruckreaktor überführt, der mit einem Flügelrührer ausgerustet ist. Dieser Stahldruckreaktor wird auf eine Innentemperatur von 100°C geheizt, wobei sich ein Innendruck von 0.6 MPa einstellt. Diesem Reaktor werden stündlich 874 g Reaktionsgemisch zugeführt, und dieselbe Menge Reaktionsgemisch wird entnommen.

Das entnommene Reaktionsgemisch wird, wie im Beispiel 1 beschrieben, mit den entsprechenden Mengen Schwefelsäure protoniert. Nach Abtrennen des Natriumsulfats durch Filtration und Waschen mit Methanol werden Filtrat und Waschmethanol vereinigt und destilliert. Vorteilhaft werden zunächst die Leichtsieder mit Hilfe eines Rotationsverdampfers oder eines Dünnschichtverdampfers abgetrennt. Durch Destillation des Rückstandes über eine 20 cm lange mit Raschig-Ringen gefüllte Kolonne erhalt man 332 g/h 2-Acetyl-gamma-butyrolacton (Reinheit nach GC 99%) was einer Ausbeute von 86 % d.Th. entspricht, bezogen auf eingesetztes gamma-Butyrolacton.

### Beispiel 3

In dem 2 l Glasreaktor der vorhergehenden Beispiele werden 500 ml Toluol als Grundfüllung vorgelegt und auf 60°C erwärmt. Innerhalb von 60 min werden dann über die Dosierpumpe ein Gemisch von 258 g gamma-Butyrolacton und 502 g Ethylacetat sowie über eine Laborzahnradpumpe eine Suspension von 244 g Natriummethylat-Pulver in 200 g Toluol zugeführt. Die Suspension von Natriummethylat-Pulver in Toluol wird in einer gesonderten Vorlage gut durchmischt. Der Reaktor wird anfangs gekühlt. so daß eine Reaktorinnentemperatur von 60°C eingehalten wird. Nach 1 h wird mit der Abnahme von Reaktionsgemische begonnen, während die Zuführung von 258 g/h gamma-Butyrolacton im Gemisch mit 502 g Ethylacetat sowie von 244 g/h Natriummethylat-Pulver suspendiert in 200 g/h Toluol fortgesetzt wird. Die mittlere Verweilzeit beträgt etwa 1 h Das dem Reaktor entnommene Gemisch wird in den im Beispiel 2 beschriebenen zweiten Stahldruckzylinder überführt. Dessen Innentemperatur betragt wiederum 100°C, der Innendruck wiederum 0,6 MPa.

Das aus dem Stahldruckzylinder austretende Reaktionsgemisch (1 204 g/h) wird auf die in Beispiel 1 beschriebene Weise protoniert. Man filtriert das Natriumsulfat ab, wäscht es mit Methanol, entfernt zunachst die Leichtsieder in einem Umlaufverdampfer und erhält durch Destillation des Rückstandes über die 2o cm lange mit Raschig-Ringen gefüllte Kolonne. 338 g 2-Acetyl-gamma-butyrolacton (Reinheit nach GC 99%), entsprechend einer Ausbeute von 88 % d.Th., bezogen auf eingesetztes gamma-Butyrolacton.

### Beispiel 4

Es wird verfahren wie in Beispiel 1, die Protonierung wird jedoch mit 50 %iger Phosphorsäure durchgeführt. Man erhält durch Destillation 464 g/h 2-Acetyl-gamma-butyrolacton (Reinheit nach GC 98.5%), entsprechend einer Ausbeute von 91 % d.Th., bezogen auf eingesetztes gamma-Butyrolakton.

### Beispiel 5

Es wird verfahren wie in Beispiel 1, die Protonierung wird jedoch mit 60 %iger Essigsäure durchgeführt. Man erhält durch Destillation 458 g/h 2-Acetyl-gamma-butyrolacton (Reinheit nach GC 98.5%), entsprechend einer Ausbeute von 88 % d.Th., bezogen auf eingesetztes gamma-Butyrolakton.

## Patentansprüche

1. Verfahren zur Herstellung von 2-Acetyl-gamma-butyrolacton aus gamma-Butyrolacton und einem Essigsäureester in einer Kondensationsreaktion unter Mitwirkung eines stark basischen Mittels mit nachfolgender Protonierung des zunächst entstehenden Enolats, **dadurch gekennzeichnet, daß** man das gamma-Butyrolacton, den Essigsäureester und den stark basischen Stoff in einem Verhältnis von 1,0 bis 6.0 Molteilen Essigsäureester und 0,9 bis 1,6 Molteilen des stark basischen Stoffs je Molteil gamma-Butyrolacton kontinuierlich einer Reaktionszone zuführt, in der die Kondensationsreaktion stattfindet und aus der anteilsweise oder kontinuierlich Reaktionsgemisch abgezogen und protoniert wird, und wobei die mittlere Verweilzeit in der Reaktionszone 5 Minuten bis 30 Stunden beträgt.

2. Verfahren nach Anspruch 1, **dadurch gekennzeichnet, daß** die Temperatur in der Reaktionszone 20 bis 160°C, vorteilhaft 30 bis 140°C beträgt.

3. Verfahren nach einem der Ansprüche 1 bis 2, **dadurch gekennzeichnet, daß** man die Reaktionszone unterteilt, indem man in einer Reaktorkaskade arbeitet.

4. Verfahren nach einem der Ansprüche 1 bis 3, **dadurch gekennzeichnet, daß** man als Essigsäureester einen Ester der Essigsäure mit einem Alkanol mit 1 bis 4 Kohlenstoffatomen verwendet.

5. Verfahren nach einem der Ansprüche 1 bis 4, **dadurch gekennzeichnet, daß** man als Essigsäureester Methylacetat verwendet.

6. Verfahren nach einem der Ansprüche 1 bis 5, **dadurch gekennzeichnet, daß** man als stark basischen Stoff ein Alkalialkoholat eines Alkanols mit 1 bis 4 Kohlenstoffatomen verwendet.

7. Verfahren nach einem der Ansprüche 1 bis 6, **dadurch gekennzeichnet, daß** man als stark basischen Stoff Natriummethylat verwendet.

8. Verfahren nach einem der Ansprüche 6 oder 7, **dadurch gekennzeichnet, daß** man den stark basischen Stoff in feinteiliger Form, gegebenenfalls in einer Teilmenge des Essigsäureesters oder einem inerten, nicht polaren Lösungsmittel suspendiert, zuführt.

9. Verfahren nach einem der Ansprüche 1 bis 8, **dadurch gekennzeichnet, daß** man die Protonierung kontinuierlich mit einer anorganischen Säure, einer organischen Säure oder einem organischen Säureanhydrid sowie bei einer Temperatur von -5 bis +50°C und einem pH von 4 bis 7 durchführt.

10. Verfahren nach einem der Ansprüche 1 bis 8, **dadurch gekennzeichnet, daß** man die Protonierung kontinuierlich mit Schwefelsäure bei einer Temperatur von 10 bis 30°C und einem pH von 5 bis 6 durchführt.

## Claims

1. A method for preparing 2-acetyl-γ-butyrolactone from γ-butyrolactone and an acetic acid ester in a condensation reaction in which a strongly basic agent also takes part, followed by protonation of the initially formed enolate, **characterized in that** the γ-butyrolactone, the acetic acid ester and the strongly basic substance, in a ratio of from 1.0 to 6.0 mols of acetic acid ester and from 0.9 to 1.6 mols of the strongly basic substance per mol of γ-butyrolactone, are fed continuously to a reaction zone in which the condensation reaction takes place and from which the reaction mixture is drawn off batchwise or continuously and protonated, the mean residence time in the reaction zone being from 5 minutes to 30 hours.

2. A method according to claim 1, **characterized in that** the temperature in the reaction zone is from 20 to 160°C, advantageously from 30 to 140°C.

3. A method according to either one of claims 1 and 2, **characterized in that** the reaction zone is subdivided by the process taking place in a reactor cascade.

4. A method according to any one of claims 1 to 3, **characterized in that** the acetic acid ester used is an ester of acetic acid with an alkanol having from 1 to 4 carbon atoms.

5. A method according to any one of claims 1 to 4, **characterized in that** the acetic acid ester used is methyl acetate.

6. A method according to any one of claims 1 to 5, **characterized in that** the strongly basic substance used is an alkali metal alcoholate of an alkanol having from 1 to 4 carbon atoms.

7. A method according to any one of claims 1 to 6, **characterized in that** the strongly basic substance used is sodium methoxide.

8. A method according to either of claims 6 and 7, **characterized in that** the strongly basic substance is fed in a finely disperse form, suspended or not suspended in an aliquot of the acetic acid ester or an inert, nonpolar solvent.

9. A method according to any one of claims 1 to 8, **characterized in that** the protonation is carried out continuously by means of an inorganic acid, an organic acid or an organic acid anhydride and at a temperature of from -5 to +50°C and a pH of from 4 to 7.

10. A method according to any one of claims 1 to 8, **characterized in that** the protonation is carried out continuously with sulphuric acid at a temperature of from 10 to 30°C and a pH of from 5 to 6.

## Revendications

1. Procédé de préparation de 2-acétyl-gamma-butyrolactone, à partir de gamma-butyrolactone et d'un ester d'acide acétique dans une réaction de condensation en faisant interagir un agent fortement basique avec protonation subséquente de l'enolate qui s'est formé en premier lieu,
**caractérisé en ce qu'**
on amène la gamma-butyrolactone, l'ester d'acide acétique et la substance fortement basique dans un rapport de 1,0 à 6,0 parties en mol d'ester d'acide acétique et de 0,9 à 1,6 parties en mol de la substance fortement basique par partie en mol de gamma-butyrolactone, en continu à une zone de réaction dans laquelle la réaction de condensation a lieu, et on soutire de celle-ci graduellement ou en continu le mélange réactionnel et on le protone, le temps de séjour moyen dans la zone de réaction étant de 5 minutes à 30 heures.

2. Procédé selon la revendication 1,
**caractérisé en ce que**
la température dans la zone de réaction est de 20 à 160°C, avantageusement de 30 à 140°C.

3. Procédé selon l'une des revendications 1 à 2,
**caractérisé en ce qu'**
on subdivise la zone de réaction en travaillant dans une cascade de réacteurs.

4. Procédé selon l'une quelconque des revendications 1 à 3,
**caractérisé en ce qu'**
on utilise comme ester d'acide acétique, un ester d'acide acétique avec un alkanol ayant de 1 à 4 atomes de carbone.

5. Procédé selon l'une quelconque des revendications 1 à 4,
**caractérisé en ce qu'**
on utilise comme ester d'acide acétique, l'acétate de méthyl.

6. Procédé selon l'une quelconque des revendications 1 à 5,
**caractérisé en ce qu'**
on utilise comme substance fortement basique un alcoolate de métal alcalin d'un alkanol ayant de 1 à 4 atomes de carbone.

7. Procédé selon l'une quelconque des revendications 1 à 6,
**caractérisé en ce qu'**
on utilise comme substance fortement basique, le méthylate de sodium.

8. Procédé selon l'une des revendications 6 ou 7,
**caractérisé en ce qu'**
on amène la substance fortement basique sous forme finement divisée, le cas échéant en une quantité partielle de l'ester d'acide acétique ou mis en suspension dans un solvant inerte, non polaire.

9. Procédé selon l'une quelconque des revendications 1 à 8,
**caractérisé en ce qu'**
on effectue la protonation en continu avec un acide non organique, un acide organique ou un anhydride d'acide organique, à une température allant de -5°C à +50°C et à un pH de 4 à 7.

10. Procédé selon quelconque des revendications 1 à 8,
**caractérisé en ce qu'**
on effectue la protonation en continu avec de l'acide sulfurique à une température de 10 à 30°C et à un pH de 5 à 6.
